# EUROPEAN PATENT SPECIFICATION

(11) **EP 1 337 575 B1**
(45) Date of publication and mention of the grant of the patent: **29.08.2007**
(21) Application number: 01998581.1
(22) Date of filing: 26.11.2001
(51) Int. Cl.: C08G 69/14, C08G 69/16, C08G 69/22, C07D 223/10, C07D 201/04

(54) **MODIFIED POLYLACTAM**
MODIFIZIERTES POLYLACTAM
POLYLACTAME MODIFIE

(30) Priority: 30.11.2000 EP 00126193
(43) Date of publication of application: 27.08.2003
(73) Proprietor: Koninklijke DSM N.V., 6411 TE Heerlen (NL)
(72) Inventor: NIJENHUIS, Atze, Jan, NL-6132 HB Sittard (NL)
(74) Representative: Mooij, Johannes Jacobus
(86) International application number: PCT/NL2001/000859
(87) International publication number: WO 2002/044246

(56) References cited:
- US-A- 3 228 759
- US-A- 3 950 311
- US-A- 4 579 762
- US-A- 5 164 261

## Description

The invention relates to a modified polylactam containing recurring units of lactam and minor amounts of a lactam derivative. The invention further relates to a process for the preparation of the modified polylactam, to the use of the modified polylactam, to fibres made from the modified polylactam and to the lactam derivative and its production.

Polyamide-6 can be made suitable for differential dyeing by introducing sulfonate groups in the polymer. This makes it possible to dye the fibre using both cationic and anionic dyes. These fibres also have anti-stain properties, which means that it is difficult for acid-based impurities to attach themselves to these fibres. Such polyamides-6 are mainly used for the production of carpet yarns, but also for fibres, paper coatings and glues.

US 5,164,261 describes shaped articles, such as fibres, formed of a polyamide formed by salt-blending the polyamide precursor salt with a cationic dye modifier, such as the dimethyl ester of 5-sulfoisophthalic acid, followed by polymerisation. The articles are rendered resistant to staining by acid dyes at ambient conditions either by adding an acid dye to the polymer melt or by dyeing with an acid dye from an acid dyebath at pH 2 to 7 and 60°C to 100°C so that the shaped article contains from at least 0.0048 and preferably at least 0.0096 wt % of an add dyestuff. In a preferred aspect of the invention, the shaped articles are oriented, crimped, heat-set fibres for use as the facing in carpets.

From the state of the art of US 3,950,311 it is known to introduce a sulfonate group by using an alkali metal salt of 5-sulfoisophthalic acid in combination with hexamethylenediamine (hereinafter referred as Li-sip system). For this purpose, the lithium or sodium salts can be used. These compounds are added during the polymerisation of polyamide-6 from caprolactam. The polymer is used to form products such as fibres or foils.

US 4,579,762 describes a similar process only using an alkali metal salt of 5-sulfoisophthalic acid without hexamethylenediamine.

However, the use of these compounds greatly affects the lactam polymerisation and results in several complications during the polymerisation. The reason for that is, that the carboxy groups and the amino groups of the additives interfere with the lactam and the polyamide chains. So the compounds act as bifunctional chain-stoppers and influence the molecular weight of the resulting polyamide. Furthermore, the solubility of the additives, the affects on the acidity and the balance between the amount of dicarboxylic acids and diamines are factors that complicate the polymerisation and often cause problems. In addition, the thermal properties of the polyamide are affected, the melting point is lowered and interference with the crystallisation of the polyamide occurs.

Therefore, it was the object of the present invention to prepare a modified polylactam with a new additive, which interferes with the lactam polymerisation to a lesser extent and adds functionality to the resulting modified polylactam.

This object of the invention is achieved by a modified polylactam containing recurring units of lactam and minor amounts of a lactam derivative having one or more non-polymerisable functional groups directly or indirectly attached to the carbon atoms of the lactam ring and wherein the functional group comprises a heteroatom.

The term "heteroatom" as used within the description covers all atoms except carbon atom and hydrogen atom. It is preferred that the heteroatom is selected from the group consisting of N-atom, O-atom, P-atom, S-atom, halogen-atom. Most preferred are N-atom, O-atom, P-atom, S-atom. The heteroatom containing functional groups can be further substituted with carbon or hydrogen containing groups, preferably alkyl-groups.

The term "functional group" as used within the description is defined as a group that actively takes part in stabilization, dyeability and flame retardancy of the resulting polymer.

In a preferred embodiment the lactam or the lactam derivative is selected from the group consisting of caprolactam, lauryllactam, caprylolactam or mixtures thereof. In a most preferred embodiment the lactam and the lactam derivative are both caprolactam, lauryllactam, caprylolactam, so that the resulting polylactam is made from caprolactam and modified caprolactam or from lauryllactam and modified lauryllactam or from caprylolactam and modified caprylolactam.

In a further preferred embodiment the lactam derivative contains a substituted or unsubstituted phenyl group between the functional group and the lactam group. Preferably the lactam derivative is substituted by a group according to formula I 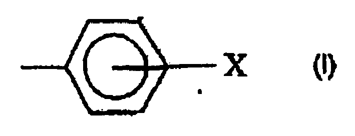 wherein X is a -SO₃H group, a -SO₂NH₂ group, a OH group, a phosphate group, a NHR₁ group or a OR₂ group or salts thereof and R₁, R₂ independently are a C₁-C₄ alkyl group, a cycloalkyl group, or a phenyl group, and wherein the phenyl ring can be substituted at each position of the carbon atom. As salts monovalent, bivalent or trivalent salts can be used, preferably salts selected from the group consisting of U, Na, K, Cu, Al, Mg, Zn, Ca, Sn, Fe salts or mixtures thereof. Most preferred are alkali metal salts, for example Li-salt, Na-salt, K-salt.

Further examples for groups substituting the lactam derivative are selected from t-butyl phenol, phenyl sulfonate, phenyl sulfonamide, phenyl phosphate, phosphate, phenol, and salts thereof.

In a most preferred embodiment the lactam derivative is substituted by a group of the formula 11 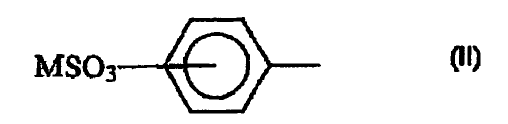 wherein one or more -SO₃M groups are present at the phenyl ring and M is lithium, sodium or potassium, wherein the caprolactam derivative having the formula III, 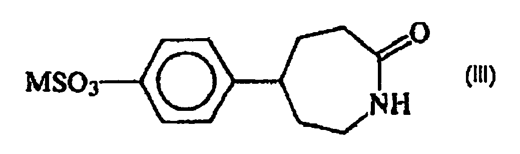 wherein M is lithium, sodium, or potassium, is mostly preferred.

As the functional group is not polymerisable the modified polylactam can contain up to 20 mol-%, preferably 0.001 to 20 mol-%, of the lactam derivative. These lactam derivative can be added during polymerisation without disturbing the polymerisation process and without a risk of gel formation.

The advantage of these compounds is, that they influence the polymerisation process to a lesser extent than the known systems of the state of the art. Thus the molecular weight and the molecular weight distribution is hardly changed. Furthermore, they do not interfere with the N-group balance and do not affect the acidity of the lactam melt.

The modified polylactam of the invention is prepared in the presence of the lactam derivative in the usual way. The polymerisation procedure incorporating the new lactam derivatives is exactly the same as for the normal lactam polymerisation. The resulting modified polylactam has essentially the same molecular weight as the normal polylactam. Furthermore, the thermal properties of the polymer are less affected in comparison with a polylactam obtained using the dicarboxylic acid/diamine system, for example with the lithium salt of 5-sulfoisophthalic acid and hexamethylenediamine.

The modified polylactam can be subjected to a postcondensation step to raise the M_{w}. Before the postcondensation step the modified polylactam has a M_{w} of 2000 to 35000, preferably 20000 to 30000 and most preferably 20000 to 25000. After postcondensation the modified polylactam has a M_{w} of 30000 to 60000, preferably 40000 to 60000 and most preferably 45000 to 55000.

The modified polylactam is used for fibres (e.g. carpet yarns), paper coatings, glues and engeneering plastics. Fibres made from the modified polylactam can further comprise usual polylactam. In this case the modified polylactam is incorporated in the polylactam by using a masterbatch of the modified polylactam, which is mixed with the regular polylactam.

The invention further relates to the new lactam derivative taking actively part in stabilisation, dyeability, and flame retardancy of the polylactam without affecting the molecular weight and the molecular weight distribution of the resulting polylactam. The most preferred lactam derivative is the caprolactam derivative having the formula III, 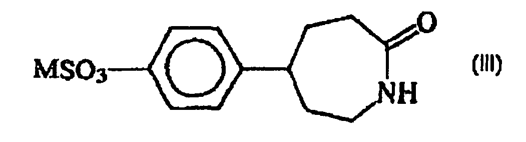 wherein M is lithium, sodium, or potassium.

The lactam derivative can be produced in the usual way by the following steps:
a. oximation reaction of a substituted cycloketone to the hydroxylimine of the ketone,
b. contacting with concentrated sulfuric acid to affect Beckmann rearrangement of hydroxylimine.

In the case the lactam derivative is the compound according to formula III a further advantage is that the compound can be obtained using the normal caprolactam synthesis starting from phenyl-cyclohexanone wherein the Beckmann rearrangement and the sulfonisation is made in one step according to the following procedure:
a. oximation reaction of phenyl cyclohexanone to the hydroxylimine of phenyl cyclohexanone,
b. contacting with concentrated sulfuric acid to affect Beckmann rearrangement of hydroxylimine of phenyl cyclohexanone and sulfonisation of phenyl ring in one step to give sulfonated phenyl caprolactam,
c. addition of alkalihydroxide and reaction of the sulfonated phenyl caprolactam to the alkali salt of sulfonated phenyl caprolactam.

Thus it is possible to produce the new additives in a usual caprolactam producing facility.

The new lactam derivatives according to the invention have a number of advantages over the existing systems of the state of the art using dicarboxylic acid salts and diamines. The lactam derivative does hardly interfere with the lactam polymerisation during the production of polylactam. As can be seen from the examples, a good incorporation of the lactam derivative takes place during the polylactam polymerisation. The use of the lactam derivative according to the invention has only a little effect on the molecular weight and molecular weight distribution compared to the known systems. The effect of the lactam derivative according to the invention on the thermal properties of the polymer is smaller than that of the known systems of the state of the art. Moreover, the use of the lactam derivative in the modified polylactam results in a higher melt viscosity at the same molecular weight in comparison with unmodified polylactam, which does not contain this additive.

A further finding is, that the lactam derivative containing polymers, have a higher zero shear melt viscosity. The increase of the zero shear melt viscosity is at least 5 %, preferably at least 10 % to 15 %, and most preferably at least 20 %. This is advantageous for blow moulding or film forming applications.

Therefore, the new lactam derivative according to the invention is a good alternative for the known additive systems containing dicarboxylic acid salts and diamines.

The invention will be further described on the basis of the following examples without being limited thereto.

### Example 1

### Monomer synthesis of the lithium salt of 5-(1-(4-sulfonic acid)-phenyl) -hexahydro-2H- azepine-2-one (5)

The starting material was 4-phenyl-cyclohexanone (1) and the process is described as follows:

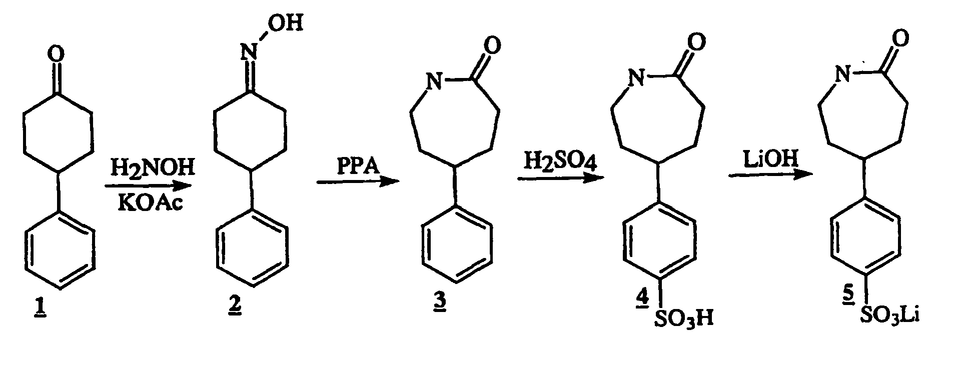

### Hydroxylimine of 4-phenyl-cyclohexanone (2)

4-phenyl-cyclohexanone (1) (103 g, 0.59 mole) was dissolved in methanol (1L). This mixture was cooled to 0°C, after which potassium acetate (173 g) and hydroxylammonium chloride (73 g) were added. The reaction mixture was then stirred for 16 hours upon which the temperature slowly rose to room temperature. Subsequently, the methanol was evaporated and the residue poured out into 1.5 L water and stirred for 10 min. The solid was removed by filtration, washed with water and dried. This resulted in 114 g of compound 2 (quantitative yield) being isolated as a white solid that was pure enough for the next step.

### 4-phenyl-hexahydro-2H-azepine-2-one (3)

The non-purified hydroxylimine 2 (108 g, max. 0.56 mole) was added in a short time to a mechanically stirred mixture of p-xylene (300 mL) and polyphosphoric acid (300 g). Subsequently, another 300 mL p-xylene was added and the mixture was heated for 3 hours until the boiling point was reached. Afterwards, the mixture was cooled to room temperature and 500 mL water was added with adequate stirring. This was followed by extraction with 1 L chloroform. The organic layer was washed with water, dried (Na₂SO₄) and evaporated. The product was recrystallised from approx. 600 mL ethanol so that 83.1 g (0.44 mole, 75 %) (2 steps from 1) of compound 3 could be isolated as a white solid.

### 5-(1-(4-sulfonic acid) -phenyl) -hexahydro-2H-azepine-2-one (4)

Phenyl caprolactam 3 (54.2 g, 287 mmol) was added in portions to 150 ml 96% sulphuric acid. During the addition, the temperature of the mixture rose to 55°C. After everything had been added (in approximately 15 minutes) the mixture was stirred for 45 minutes at 60-65°C. Next, the reaction mixture was poured into 750 mL ice water and stirred for 30 minutes, the precipitate formed being filtered off and washed with methanol. After drying, the crude yield was 55.1 g. The filtrate was left to stand for a weekend, upon which precipitate was again formed, which was filtered off and dried, so that a second portion of 14.7 g could be isolated. The two portions were combined, brought to the boil in methanol for a short time and cooled to room temperature. The precipitate was filtered, washed twice with methanol and dried, so that 67 g (249 mmol, 87%) of pure sulfonic acid 4 could be isolated.

### Lithium salt of 5-(1-(4-sulfonic acid)-phenyl)-hexahydro-2H-azepine-2-one (5)

Sulfonic acid 4 (75,5 g, 281 mmol) was stirred in 400 ml water. Subsequently, LiOH H₂O (11.8g, 281 mmol) was added and the mixture was heated to 50°C so that a clear solution was formed. 200 mL water was evaporated and the residue was added to 200 mL acetone with vigorous stirring. The precipitate formed was filtered off, washed with acetone and dried so that 47.9 g (174 mmol, 62%) pure Li-salt 5 could be isolated. Expected elementary analysis: C 52.36%, H 5.13%, N 5.09%, U 2.52%. Found: C 51.81%, H 5.06%, N 4.93%, Li 2.46%. (Purity on basis of U and N is 97%)

### Example 1a

### Monomer synthesis of the lithium salt of 5-(1-(4-sulfonic acid) -phenyl) -hexahydro-2H-azepine-2-one (5) (alternative route)

The process for the preparation of compound (5) is made in the same way as in example 1 with the difference that compound (2) is contacted with concentrated sulfuric acid to affect Beckmann rearrangement of hydroxylimine of phenyl cyclohexanone and sulfonisation of phenyl ring in one step to give sulfonated phenyl caprolactam (4).

### Example 2

### Polymerisation of ε- caprolactam to modified polycaprolactam

To the mixture of caprolactam, aminocaproic acid and water 2.2 wt-% of the lithium salt of 5-(1-(4-sulfonic acid) -phenyl) -hexahydro- 2H- azepine-2-one (hereinafter referred as Li-salt) was added after which the normal polymerisation and treatment procedure for the production of polycaprolactam was carried out.

### Example 3

### Polymerisability of the lithium salt of 5-(1-(4-sulfonic acid) -phenyl) -hexahydro-2H-azepine-2-one

It was investigated whether the Li-salt was incorporated during the polymerisation in the modified polycaprolactam. This was done by determining the concentrations of sulphur by X-ray fluorescence in the washed polymer. Non-polymerised Li-salt is water-soluble and is therefore removed during the washing step. It was found that the amount of the Li-salt incorporated in the polymer was 90%.

**Table 1: Incorporation during polymerisation of caprolactam with 2.2 wt-% Li-salt**

| | found | theory |
|---|---|---|
| Sulphur | 0.21 (± 0.02) wt-% | 0.22 wt-% |

Table 1 shows that the sulphur content found is very close to the theoretical value. On the basis of the sulphur content, Li-salt has the same reactivity relative to the polymerisation as caprolactam.

### Example 4

### Molecular weight and rheology of modified polycaprolactam versus blank polycaprolactam

The molecular weight and the rheology of the modified polycaprolactam was measured and compared with blank polycaprolactam.

**Table 2: Influence of Li-salt on the molecular weight and the rheology**

| | SEC-DV | | | | |
|---|---|---|---|---|---|
| | Mw/Mn | Mw (x10³) | Mz/Mw | [η]_{HCOOH} dl/g | η _{0 230C} Pas |
| as-polymerised | | | | | |
| | | | | | |
| blank polycaprolactam (comparison) | 2.2 | 25 | 1.6 | 1.05 | 5.9x10² |
| polycaprolactam with 0.08 meq/g Li-salt (invention) | 1.9 | 22 | 1.6 | 0.89 | 7.2x10² |
| | | | | | |
| Post-condensed | | | | | |
| | | | | | |
| blank polycaprolactam (comparison) | 2.2 | 52 | 1.9 | 1.68 | 1.3x10⁴ |
| polycaprolactam with 0.08 meq/g Li-salt (invention) | 2.0 | 49 | 1.8 | 1.57 | 1.4x10⁴ |

- SEC-DV: = size exclusion chromatography-differential viscosimetry
- Mw/Mn: = average molecular weight/number average molecular weight
- Mw: = average molecular weight
- Mz/Mw: = viscosity average molecular weight/ average molecular weight
- [η]_{HCOOH}: = intrinsic viscosity measured in formic acid
- η _{0 230C}: = zero shear melt viscosity
- Pas: = Pascalseconds
- meq: = milliequivalents

From Table 2 it is concluded that the addition of Li-salt has only very little effect on the molecular weight and the molecular weight distribution of the polymer. The SEC-DV results indicate that there is a small decrease in the molecular weight it is evident from the fact that the Li-salt does not function as a chain stopper (causes non-reactive chain ends) that the polymer can be subjected to post-condensation up to a very high molecular weight, just like the blank polycaprolactam. It should be noted that after post-condensation the differences in molecular weight are within the reproducibility of the polymerisation experiments.

A further finding is, that the Li-salt containing polymers have a higher zero shear melt viscosity. The increase of the zero shear melt viscosity in the present example is about 18 %. This is advantageous for blow moulding or film forming applications.

### Example 5

### Thermal properties of modified polycaprolactam versus blank polycaprolactam

The thermal properties of the modified polycaprolactam of the invention were measured and compared with blank polycaprolactam. Table 3 shows the results.

**Table 3: Influence of comonomers on thermal properties**

| | Tm₂ (°C) | ΔHₕ₂ (J/g) | Tcc (°C) | ΔHc₁ (J/g) | Tg (°C) |
|---|---|---|---|---|---|
| as-polymerised | | | | | |
| | | | | | |
| blank polycaprolactam (comparison) | 218.8 | 77.4 | 171.1 | 73.5 | 44 |
| polycaprolactam with 0.08 meq/g Li-salt (invention) | 216.0 | 65.7 | 164.6 | 66.2 | 45 |
| | | | | | |
| polycaprolactam with 0.08 meq/g Li-sip + 0.06 meq/g HMDA (comparison) | 212.8 | 64.2 | 160.4 | 62.1 | 63 |
| | | | | | |
| post-condensed | | | | | |
| | | | | | |
| blank polycaprolactam (comparison) | 220.2 | 76.3 | 167.7 | 76.3 | 56 |
| polycaprolactam with 0.08 meq/g Li-salt (invention) | 215.8 | 74.3 | 161.9 | 71.3 | 48 |

As evident from Table 3, the incorporation of Li-salt reduces the melting temperature (Tm₂), the crystallisation temperature during cooling (Tcc), and the melting heat formed during both melting and cooling (ΔHₕ₂, ΔHc₁). Still, there is a clear improvement in these properties compared to the known system (lithium salt of 5-sulfoisophthalic acid/Hexamethylenediamine hereinafter referred as Li-sip system) as currently in commercial use. Compared to blank polycaprolactams, post-condensation increases the difference in Tm₂, Tcc and Tg, whereas the difference in melting and crystallisation heat decreases.

## Claims

1. Modified polylactam containing recurring units of lactam and minor amounts of a lactam derivative having one or more non-polymerisable functional groups directly or indirectly attached to the carbon atoms of the lactam ring and wherein the functional group comprises a heteroatom.

2. Modified polylactam according to claim 1, **characterised in that** the lactam or the lactam derivative is selected from the group consisting of caprolactam, lauryllactam, caprylolactam or mixtures thereof.

3. Modified polylactam according to claim 1 or 2, **characterised in that** the lactam derivative contains a substituted or unsubstituted phenyl group between the functional group and the lactam group.

4. Modified polylactam according to claims 1 to 3, **characterised in that** the lactam derivative is substituted by a group according to formula I 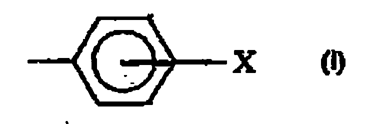 wherein X is a -SO₃H group, a -SO₂NH₂ group, a OH group, a phosphate group, a NHR₁ group or a OR₂ group and salts thereof and R₁, R₂, independently are a C₁-C₄ alkyl group, a cycloalkyl group, or a phenyl group, and wherein the phenyl ring can be substituted at each position of the carbon atom.

5. Modified polylactam according to claims 1 to 4, **characterised in that** the lactam derivative is substituted by groups selected from t-butyl phenol, phenyl sulfonate, phenyl sulfonamide, phenyl phosphate, phosphate, phenol, and salts thereof.

6. Modified polylactam according to claims 1 to 5, **characterised in that** the lactam derivative is substituted by a group of the formula II 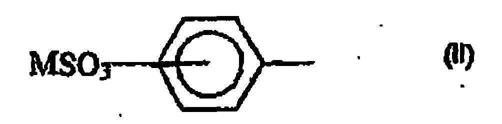 wherein one or more -SO₃M groups are present at the phenyl ring and M is lithium, sodium or potassium.

7. Modified polylactam according to claims 1 to 6, **characterised in that** the lactam derivative is a caprolactam derivative having the formula III, 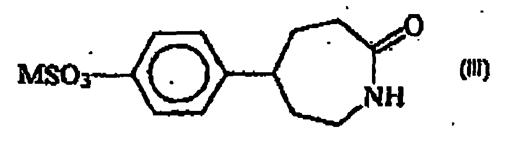 wherein M is lithium, sodium, or potassium.

8. Modified polylactam according to claims 1 to 7, **characterised in that** it contains 0.001 to 20 mol-% of the lactam derivative.

9. Process for the preparation of a modified polylactam according to claims 1 to 8, **characterised in that** the lactam polymerisation is carried out in the presence of a functionalised lactam derivative as described in claims 1 to 8.

10. Process according to claim 9, **characterised in that** the modified polylactam is subjected to a postcondensation.

11. Use of the modified polylactam according to claims 1 to 8 for fibres, paper coatings, glues, engeneering plastics, blow moulding and films.

12. Fibres comprising the modified polylactam according to claims 1 to 8.

13. Fibres according to claim 11 further comprising polylactam.

14. Lactam derivative as described in claims 4 to 8.

15. Use of the lactam derivative according to claim 14 for adding functionality to polylactam without affecting the molecular weight and the molecular weightdistribution of the resulting polylactam.

16. Process for the manufacture of the lactam derivative according to claims 4 to 7 by the following steps:
a. oximation reaction of a substituted cycloketone to the hydroxylimine of the ketone,
b. contacting with concentrated sulfuric acid to affect Beckmann rearrangement of hydroxylimine.

17. Process for the preparation of the lactam derivative according to claim 6 or 7, **characterised by** the following steps:
a. oximation reaction of phenyl cyclohexanone to the hydroxylimine of phenyl cyclohexanone,
b. contacting with concentrated sulfuric acid to affect Beckmann rearrangement of hydroxylimine of phenyl cyclohexanone and sulfonisation of phenyl ring in one step to give sulfonated phenyl caprolactam,
c. addition of alkalihydroxide and reaction of the sulfonated phenyl caprolactam to the alkali salt of sulfonated phenyl caprolactam.

## Patentansprüche

1. Modifiziertes Polylactam, enthaltend wiederkehrende Lactameinheiten und geringe Anteile eines Lactamderivats mit mindestens einer direkt oder indirekt mit den Kohlenstoffatomen des Lactamrings verbundenen nicht polyreaktionsfähigen funktionellen Gruppe, wobei die funktionelle Gruppe ein Heteroatom enthält.

2. Modifiziertes Polylactam nach Anspruch 1, **dadurch gekennzeichnet, daß** das Lactam bzw. das Lactamderivat ausgewählt ist aus der Gruppe, bestehend aus Caprolactam, Lauryllactam, Caprylolactam und deren Mischungen.

3. Modifiziertes Polylactam nach Anspruch 1 oder 2, **dadurch gekennzeichnet, daß** das Lactamderivat zwischen funktioneller Gruppe und Lactamgruppe eine gegebenenfalls substituierte Phenylgruppe enthält.

4. Modifiziertes Polylactam nach den Ansprüchen 1 bis 3, **dadurch gekennzeichnet, daß** das Lactamderivat substituiert ist durch eine Gruppe gemäß der Formel I 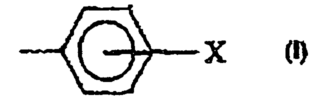 wobei X eine -SO₃H-Gruppe, eine -SO₂NH₂-Gruppe, eine OH-Gruppe, eine Phosphatgruppe, eine NHR₁-Gruppe oder eine OR₂-Gruppe und Salze davon und R₁ und R₂ unabhängig voneinander eine C₁-C₄-Alkylgruppe, eine Cycloalkylgruppe oder eine Phenylgruppe bedeuten und wobei der Phenylring an beliebiger Position des Kohlenstoffatoms substituiert sein kann.

5. Modifiziertes Polylactam nach den Ansprüchen 1 bis 4, **dadurch gekennzeichnet, daß** das Lactamderivat substituiert ist durch Gruppen, ausgewählt aus t-Butylphenol, Phenylsulfonat, Phenylsulfonamid, Phenylphosphat, Phosphat, Phenol und deren Salze.

6. Modifiziertes Polylactam nach den Ansprüchen 1 bis 5, **dadurch gekennzeichnet, daß** das Lactamderivat substituiert ist durch eine Gruppe der Formel II 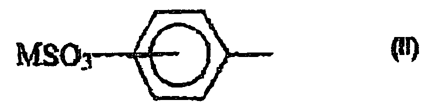 wobei eine oder mehrere der -SO₃M-Gruppen am Phenylring stehen und M Lithium, Natrium oder Kalium bedeutet.

7. Modifiziertes Polylactam nach den Ansprüchen 1 bis 6, **dadurch gekennzeichnet, daß** es sich bei dem Lactamderivat um ein Caprolactamderivat mit der Formel III 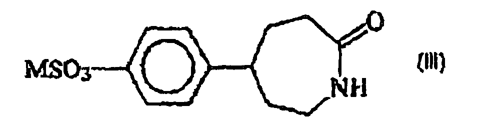 handelt, wobei M Lithium, Natrium oder Kalium bedeutet.

8. Modifiziertes Polylactam nach den Ansprüchen 1 bis 7, **dadurch gekennzeichnet, daß** es 0,001 bis 20 mol-% des Lactamderivats enthält.

9. Verfahren zur Herstellung eines modifizierten Polylactams gemäß den Ansprüchen 1 bis 8, **dadurch gekennzeichnet, daß** die Lactampolymerisation in Gegenwart eines funktionalisierten Lactamderivats gemäß den Ansprüchen 1 bis 8 erfolgt.

10. Verfahren nach Anspruch 9, **dadurch gekennzeichnet, daß** man das modifizierte Polylactam einer Nachkondensation unterwirft.

11. Verbindung des modifizierten Polylactams gemäß den Ansprüchen 1 bis 8 für Fasern, Papierstreichmassen, Klebmittel, technische Kunststoffe, Blasformen und Folien.

12. Fasern mit dem modifizierten Polylactam gemäß den Ansprüchen 1 bis 8.

13. Fasern gemäß Anspruch 11, die zusätzlich Polylactam enthalten.

14. Lactamderivat gemäß den Ansprüchen 4 bis 8.

15. Verwendung des Lactamderivats gemäß Anspruch 14 zur Ausstattung von Polylactam mit zusätzlicher Funktionalität, ohne dabei das Molekulargewicht und die Molekulargewichtsverteilung des dabei entstehenden Polylactams zu beeinflussen.

16. Verfahren zur Herstellung des Lactamderivats gemäß den Ansprüchen 4 bis 7, bei dem man
a. ein substituiertes Cycloketon zum Hydroxylimin des Ketons oximiert,
b. durch Einwirkung konzentrierter Schwefelsäure eine Beckmann'sche Umlagerung des Hydroxylimins bewirkt.

17. Verfahren zur Herstellung des Lactamderivats gemäß Anspruch 6 oder 7, bei dem man
a. Phenylcyclohexanon zum Hydroxylimin des Phenylcyclohexanons oximiert,
b. unter Einwirkung konzentrierter Schwefelsäure eine Beckmann'sche Umlagerung des Hydroxylimins von Phenylcyclohexanon und eine Sulfonierung des Phenylrings in einem Schritt bewirkt, wobei man sulfoniertes Phenylcaprolactam erhält,
c. das sulfonierte Phenylcaprolactam mit Alkalihydroxid versetzt und zum Alkalisalz des sulfonierten Phenylcaprolactams umsetzt.

## Revendications

1. Polylactame modifié renfermant des motifs récurrents de lactame et des quantités mineures d'un dérivé de lactame renfermant un ou plusieurs groupes fonctionnels non-polymérisables fixés directement ou indirectement aux atomes de carbone du noyau lactame, et dans lequel le groupe fonctionnel renferme un hétéroatome.

2. Polylactame modifié selon la revendication 1, **caractérisé en ce que** le lactame ou le dérivé de lactame est choisi parmi le groupe constitué du caprolactame, du lauryllactame, du caprylolactame ou de leurs mélanges.

3. Polylactame modifié selon la revendication 1 ou 2, **caractérisé en ce que** le dérivé de lactame renferme un groupe phényle substitué ou non-substitué entre le groupe fonctionnel et le groupe lactame.

4. Polylactame modifié selon les revendications 1 à 3, **caractérisé en ce que** le dérivé de lactame est substitué par un groupe selon la formule I 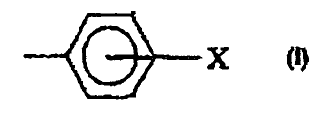 dans laquelle X est un groupe -SO₃H, un groupe -SO₂NH₂, un groupe OH, un groupe phosphate, un groupe NHR₁ ou un groupe OR₂, et leurs sels, et R₁ et R₂ sont indépendamment un groupe alkyle en C₁-C₄, un groupe cycloalkyle ou un groupe phényle, et dans laquelle le noyau phényle peut être substitué en chaque position de l'atome de carbone.

5. Polylactame modifié selon les revendications 1 à 4, **caractérisé en ce que** le dérivé de lactame est substitué par des groupes choisis parmi un groupe t-butylphénol, un groupe phénylsulfonate, un groupe phénylsulfonamide, un groupe phényl-phosphate, un groupe phosphate, un groupe phénol, et leurs sels.

6. Polylactame modifié selon les revendications 1 à 5, **caractérisé en ce que** le dérivé de lactame est substitué par un groupe de formule II 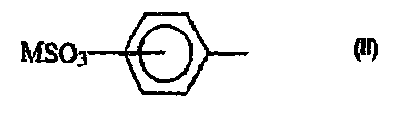 dans laquelle un ou plusieurs groupes -SO₃M sont présents au niveau du noyau phényle et M est le lithium, le sodium ou le potassium.

7. Polylactame modifié selon les revendications 1 à 6, **caractérisé en ce que** le dérivé de lactame est un dérivé de caprolactame de formule III 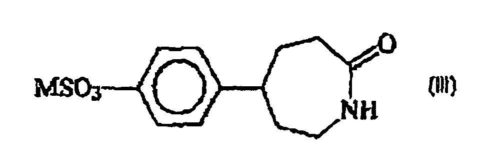 dans laquelle M est le lithium, le sodium ou le potassium.

8. Polylactame modifié selon les revendications 1 à 7, **caractérisé en ce qu'**il contient de 0,001 à 20 % en moles du dérivé de lactame.

9. Procédé de préparation d'un polylactame modifié selon les revendications 1 à 8, **caractérisé en ce que** la polymérisation du lactame est effectuée en présence d'un dérivé de lactame fonctionnalisé tel que décrit dans les revendications 1 à 8.

10. Procédé selon la revendication 9, **caractérisé en ce que** le polylactame modifié est soumis à une post-condensation.

11. Utilisation du polylactame modifié selon les revendications 1 à 8 pour des fibres, des revêtements de papier, des colles, des matières plastiques d'ingénierie, le moulage par extrusion-soufflage et des films.

12. Fibres comprenant le polylactame modifié selon les revendications 1 à 8.

13. Fibres selon la revendication 11, comprenant en outre un polylactame.

14. Dérivé de lactame tel que décrit dans les revendications 4 à 8.

15. Utilisation du dérivé de lactame selon la revendication 14 pour ajouter une fonctionnalité à un polylactame sans affecter le poids moléculaire et la distribution du poids moléculaire du polylactame résultant.

16. Procédé de fabrication du dérivé de lactame selon les revendications 4 à 7 par les étapes suivantes:
a. une réaction d'oximation d'une cyclocétone substituée en l'hydroxylimine de la cétone,
b. une mise en contact avec de l'acide sulfurique concentré pour réaliser le réarrangement de Beckmann de l'hydroxylimine.

17. Procédé de préparation du dérivé de lactame selon la revendication 6 ou 7, **caractérisé par** les étapes suivantes :
a. une réaction d'oximation de la phénylcyclohexanone en l'hydroxylimine de la phénylcyclohexanone,
b. une mise en contact avec de l'acide sulfurique concentré pour réaliser le réarrangement de Beckmann de l'hydroxylimine de la phénylcyclohexanone et la sulfonation du noyau phényle en une seule étape pour donner lieu au phényl-caprolactame sulfoné,
c. une addition d'un hydroxyde de métal alcalin et une réaction du phényl-caprolactame sulfoné pour donner lieu au sel de métal alcalin du phényl-caprolactame sulfoné.
